(19) 
**Europäisches Patentamt**
**European Patent Office**
**Office européen des brevets**

(11) **EP 4 651 039 A1**

(12) # EUROPEAN PATENT APPLICATION

(43) Date of publication:
**19.11.2025 Bulletin 2025/47**

(21) Application number: **25175180.6**

(22) Date of filing: **08.05.2025**

(51) International Patent Classification (IPC):
**G06N 10/20** (2022.01)    **G06N 10/60** (2022.01)
**G06N 5/01** (2023.01)    **G06N 20/00** (2019.01)

(52) Cooperative Patent Classification (CPC):
**G06N 10/60; G06N 10/20;** G06N 5/01; G06N 20/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **15.05.2024 JP 2024079077**

(71) Applicant: **Fujitsu Limited**
**Kawasaki-shi, Kanagawa 211-8588 (JP)**

(72) Inventor: **ITO, Yuto**
**Kawasaki-shi, 211-8588 (JP)**

(74) Representative: **Haseltine Lake Kempner LLP**
**Cheapside House**
**138 Cheapside**
**London EC2V 6BJ (GB)**

(54) **COMPUTER PROGRAM, QUANTUM CHEMICAL COMPUTATION METHOD, AND INFORMATION PROCESSING APPARATUS**

(57) An information processing apparatus iterates a process of updating a value of a coefficient in a third equation and a process of searching for a ground state of a many-electron system. The third equation is obtained by adding a term, which is a product of a second equation relating to the number of electrons in the many-electron system and the coefficient, to a first equation for computing a physical quantity of the many-electron system. The search process employs a variational quantum eigensolver method and searches for the ground state of the many-electron system by setting, in the (k + 1)th search process, a final state of the many-electron system obtained in one of the first to k-th search processes as an initial state, and then changing the quantum state of the many-electron system from the initial state such that the expected value of the third equation is reduced.

FIG. 1

EP 4 651 039 A1

**Description**

FIELD

The embodiments discussed herein relate to a computer program, a quantum chemical computation method, and an information processing apparatus.

BACKGROUND

As one of the quantum chemical computations using a quantum computer, there is a computation method called a variational quantum eigensolver (VQE) method. The VQE is a method of computing an eigenvalue (for example, a minimum eigenvalue of energy) and an eigenstate of a physical quantity in a many-electron system of quantum mechanics. In the VQE, for example, an expected energy value (a minimum eigenvalue of energy) of the ground state of a molecule is computed by the following procedure.

1. Under the control of a classical computer, a trial state "$|\psi(\theta)\rangle$" in a quantum mechanical many-electron system is generated in a quantum computer ($\theta$ is a real number parameter).
2. The quantum computer computes an expected value "$\langle\psi(\theta)|H|\psi(\theta)\rangle$" of the energy in the trial state (H is a Hamiltonian).
3. Based on the computation result of the expected value of the energy, the classical computer updates the value of the parameter $\theta$ such that the expected value "$\langle\psi(\theta)|H|\psi(\theta)\rangle$" is reduced.

By the cooperative operation of the classical computer and the quantum computer, the above process is iterated until the expected value "$\langle\psi(\theta)|H|\psi(\theta)\rangle$" of the energy converges. Thus, an approximate minimum energy value and the corresponding state are obtained.

As the function form of the trial state "$|\psi(\theta)\rangle$", a function form having high computation efficiency in the quantum computer is selected. Depending on the selected function form, a state that deviates from the assumed conditions (for example, a state in which the number of electrons is incorrect) may be obtained. In this case, for example, an equation "H(p): = H + $\mu$C" ($\mu$ is a coefficient and C is the number of electrons in the state of H) obtained by adding a penalty term to an operator (Hamiltonian H) for obtaining energy is used, so as to compute VQE for obtaining a state in which the expected value of H($\mu$) is minimized.

As a computation using a penalty term in a quantum computer, for example, a hybrid algorithm for obtaining a solution of a discrete quadratic model has been proposed. A method of modifying a cost function in quantum approximation optimization has also been proposed. A technique for improving a system for processing inequality constraints in a mixed binary optimization problem on a quantum computer and significantly improving the performance of the system has also been proposed. In addition, a system that facilitates optimization of quantum-enhanced feature generation has been proposed.

Japanese National Publication of International Patent Application No. 2023-507139
Japanese National Publication of International Patent Application No. 2021-504805
U.S. Patent Application Publication No. 2021/0216897
U.S. Patent Application Publication No. 2023/0143072

When a computer performs a VQE computation using a function including a penalty term, the computer performs the VQE computation iteratively while updating the value of a coefficient of the penalty term. Therefore, by iteratively executing the VQE computation, the computation time to obtain the ground state energy value increases.

SUMMARY

In one aspect, an object of the present embodiments is to improve the efficiency of VQE computation.

In one aspect, there is provided a computer program that causes a computer to execute a process including: alternately iterating a process of updating a value of a coefficient in a third equation obtained by adding a term obtained by multiplying a second equation relating to a number of electrons in a many-electron system by the coefficient to a first equation for computing a physical quantity of the many-electron system, and a search process of searching for a ground state of the many-electron system based on a variational quantum eigensolver method by changing a quantum state of the many-electron system such that an expected value of the third equation based on the quantum state of the many-electron system is reduced, wherein the search process in a (k + 1)th iteration (k is a natural number) includes setting of a final state of the many-electron system obtained in the search process in any one of first to k-th iterations as an initial state of the many-electron system in the search process in the (k + 1)th iteration, and changing of the quantum state of the many-electron system from the initial state such that the expected value of the third equation is reduced.

BRIEF DESCRIPTION OF DRAWINGS

FIG. 1 is a diagram illustrating an example of a quantum chemical computation method according to a first embodiment;
FIG. 2 is a diagram illustrating an example of a configuration of a quantum computing system;
FIG. 3 is a diagram illustrating an example of hardware of apparatuses constituting the quantum computing system;
FIG. 4 is a diagram illustrating an example of a computation procedure of VQE using an equation to which a penalty term including a coefficient $\mu$ is added;
FIG. 5 is a block diagram illustrating an example of functions of a classical computer that performs quantum chemical computation;
FIG. 6 is a diagram illustrating an example of a method of adjusting the coefficient $\mu$ of the penalty term;
FIG. 7 is a flowchart illustrating an example of a procedure of a VQE-based quantum chemical computation process;
FIG. 8 is a diagram illustrating an example of a case where the conversion of the coefficient $\mu$ by a Newton method is not monotonic;
FIG. 9 is a diagram illustrating an example of a method of determining the initial value of a parameter $\theta$ for the next VQE computation from the results of a plurality of past VQE computations; and
FIG. 10 is a flowchart illustrating an example of a procedure of a VQE-based quantum chemical computation process according to the third embodiment.

DESCRIPTION OF EMBODIMENTS

Hereinafter, embodiments will be described with reference to the drawings. Each embodiment may be implemented by combining a plurality of embodiments within a consistent range.

[First Embodiment]

A first embodiment is a quantum chemical computation method for improving the efficiency of VQE computation.

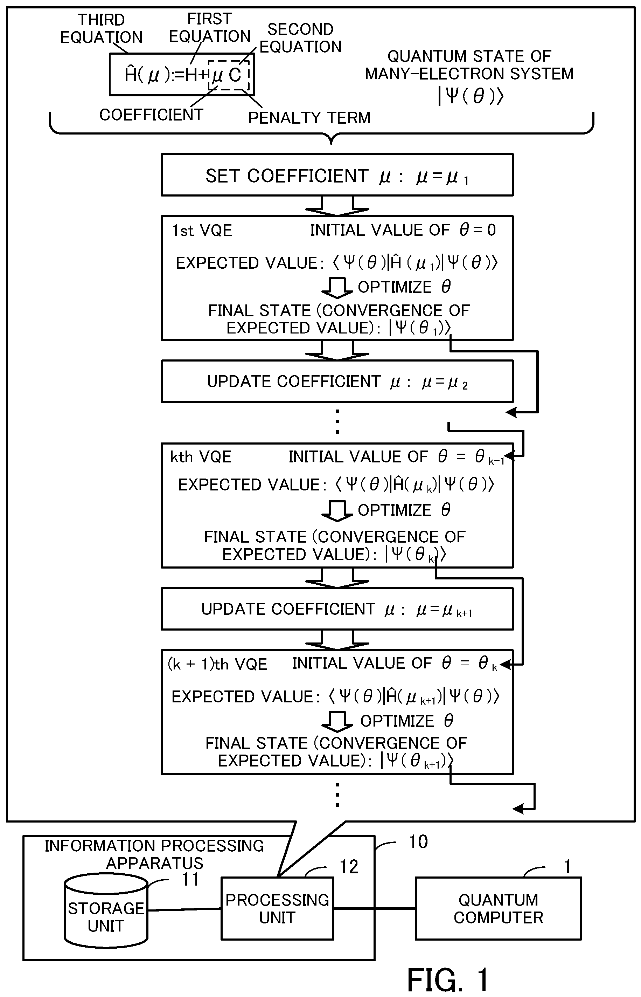

FIG. 1 is a diagram illustrating an example of a quantum chemical computation method according to the first embodiment. FIG. 1 illustrates an information processing apparatus 10 that performs a quantum chemical computation method. The information processing apparatus 10 performs quantum chemical computation in cooperation with a quantum computer 1. The information processing apparatus 10 is able to implement a quantum chemical computation method by executing, for example, a quantum chemical computation program.
The information processing apparatus 10 includes a storage unit 11 and a processing unit 12. The storage unit 11 is, for example, a memory or a storage device included in the information processing apparatus 10. The processing unit 12 is, for example, a processor or an arithmetic circuit included in the information processing apparatus 10.
The storage unit 11 stores, for example, a quantum chemical computation program and information about a many-electron system to be solved.
The processing unit 12 obtains a ground state (a state in which energy indicates a minimum value) of the many-electron system by executing the VQE. For example, the processing unit 12 defines a third equation "$\hat{H}(\mu) = H + \mu(C)$" obtained by adding a term obtained by multiplying a second equation "$C$" relating to the number of electrons in the many-electron system by a coefficient "$\mu$" to the first equation for computing a physical quantity of the many-electron system. The term obtained by multiplying the second expression relating to the number of electrons in the many-electron system by the coefficient is, for example, a penalty term for preventing the number of electrons included in the quantum state of the many-electron system in the computation process from greatly deviating from the correct number of electrons.
The quantum state of the many-electron system is represented by "$|\Psi(\theta)\rangle$". The quantum state changes according to the value of a parameter $\theta$. The parameter $\theta$ is, for example, vector data including a plurality of real values as elements.
The processing unit 12 alternately iterates a process of updating the value of the coefficient in the third equation and a search process of searching for the ground state of the many-electron system based on the VQE using the third equation. In the process of updating the value of the coefficient, the processing unit 12 updates the value of the coefficient such that, for example, the number of electrons included in the quantum state of the many-electron system approaches the correct number of electrons.
In the search process of searching for the ground state of the many-electron system, the processing unit 12 changes the quantum state of the many-electron system such that the expected value of the third equation based on the quantum state of the many-electron system is reduced. For example, the processing unit 12 computes the expected value "$\langle\Psi(\theta)|\hat{H}H|\Psi(\theta)\rangle$" of the third equation based on the quantum state of the many-electron system, and optimizes the value of the parameter $\theta$ such that the expected value is reduced. Then, when the expected value converges, the processing unit 12 obtains the quantum state of the many-electron system as the final state.

In the search process in the (k + 1)th iteration (k is a natural number) for the ground state of the many-electron system, the processing unit 12 sets the final state of the many-electron system in the search process in any one of the first to k-th iteration for the ground state of the many-electron system as the initial state of the many-electron system in the search process in the (k + 1)th iteration. Then, the processing unit 12 changes the quantum state of the many-electron system from the initial state such that the expected value of the third equation is reduced.

The processing unit 12 changes the quantum state of the many-electron system while allowing fluctuation in the number of electrons in the many-electron system, for example. Then, the processing unit 12 determines, in response to the number of electrons in the many-electron system in the final state of the many-electron system satisfying a predetermined condition, the final state of the many-electron system as the ground state of the many-electron system. The predetermined condition is, for example, a condition that the difference between the number of electrons in the final state of the many-electron system and the number of electrons originally included in the many-electron system is within an allowable range indicated by a threshold.

For example, the processing unit 12 first sets a predetermined real number value to the coefficient $\mu$ as an initial value "$\mu_1$". In the first VQE computation, the processing unit 12 sets a predetermined value to the parameter $\theta$ as an initial value. For example, the processing unit 12 sets an initial value "0" to the parameter $\theta$ (values of all elements included in the parameter $\theta$ are "0").

The processing unit 12 optimizes the value of the parameter $\theta$ such that the expected value "$\langle\Psi(\theta)|\hat{H}(\mu_1)|\Psi(\theta)\rangle$" of the third equation based on the quantum state of the many-electron system is reduced. The present embodiment assumes that the expected value has converged when the value of the parameter $\theta$ becomes "$\theta_1$". The quantum state "$|\Psi(\theta_1)\rangle$" of the many-electron system when the expected value has converged is the final state in the first VQE. There is a possibility that the number of electrons in the quantum state "$|\Psi(\theta_1)\rangle$" of the many-electron system in the final state greatly deviates from the correct number of electrons of the many-electron system.

If the number of electrons in the final state "$|\Psi(\theta_1)\rangle$" of the many-electron system in the first VQE satisfies a predetermined condition, the processing unit 12 determines that the final state "$|\Psi(\theta_1)\rangle$" is the ground state of the many-electron system. If the number of electrons in the final state "$|\Psi(\theta_1)\rangle$" of the many-electron system in the first VQE does not satisfy the predetermined condition, the processing unit 12 updates the value of the coefficient $\mu$ ($\mu = \mu_2$). Thereafter, the search for the ground state by the VQE and the update of the coefficient $\mu$ are iterated until the predetermined condition regarding the number of electrons is satisfied.

In the VQE computation, for example, the final state in the previous VQE computation is used as the initial state of the many-electron system. The quantum state of the many-electron system is determined by the value of the parameter $\theta$.

For example, the final state "$|\Psi(\theta_k)\rangle$" of the many-electron system in the k-th VQE computation is applied to the initial state of the many-electron system in the (k + 1)th VQE computation. That is, the initial value of the parameter $\theta$ in the (k + 1)th VQE is "$\theta_k$". In the k-th VQE computation, the processing unit 12 optimizes $\theta$ such that the expected value "$\langle\Psi(\theta)|\hat{H}(\mu_{k+1})|\Psi(\theta)\rangle$" obtained by the third equation for the quantum state of the many-electron system decreases from the initial state "$|\Psi(\theta k)\rangle$" of the many-electron system.

In this quantum chemical computation, the quantum state obtained as a result of the k-th VQE is used as the initial state of the (k + 1)th VQE. Accordingly, the expected value efficiently converges in the VQE computation. As a result, the efficiency of the computation in the entire quantum system computation process using the VQE is improved. That is, if the update width of the coefficient $\mu$ of the penalty term is small every time, it is considered that the quantum state that minimizes the expected value of "$\hat{H}(\mu)$" does not change greatly. Therefore, if the final state of the k-th VQE computation is set as the initial state of the (k + 1)th VQE computation, the final state is reached with a small change from the initial state in the (k + 1)th VQE computation. Therefore, the process time of the (k + 1)th VQE is shortened.

The process of setting the final state of the k-th VQE as the initial state of the (k + 1)th VQE is particularly effective when the value of the coefficient $\mu$ monotonically changes. In a case where the value of the coefficient $\mu$ does not monotonically change, there is a possibility that the computation efficiency is improved by using the final state of a VQE computation other than the immediately preceding VQE computation.

Thus, the processing unit 12 may determine an appropriate quantum state as the initial state in the (k + 1)th VQE computation based on the difference between the values of the coefficients $\mu$ used in the first to k-th VQE computations and the value of $\mu$ used in the (k + 1)th VQE computation.

For example, the processing unit 12 selects one of the values of the coefficients $\mu$ applied to the first to k-th VQE computations, based on the difference between an individual value of the coefficient $\mu$ of "$\hat{H}(\mu)$" used in the first to k-th VQE computations and the value of the coefficient $\mu$ used in the (k + 1)th VQE computation. For example, the processing unit 12 selects a value having the smallest difference from the value of the coefficient $\mu$ used in the (k + 1)th VQE computation. The processing unit 12 sets the final state of the many-electron system in the VQE executed using "$\hat{H}(\mu)$" in which the selected value is the value of the coefficient as the initial state of the many-electron system in the (k + 1)th VQE.

In this way, even in a case where the value of the coefficient $\mu$ does not monotonically change, in each VQE computation, it is possible to search for the ground state from an appropriate initial state of the many-electron system, and the efficiency of the VQE computation process is improved.

[Second Embodiment]

The second embodiment is a quantum computing system that sets, in a case where the quantum computing system iterates a VQE computation while updating a coefficient of a penalty term, a state obtained as a result of the previous VQE computation as the initial value of the qubit state in the current VQE computation.

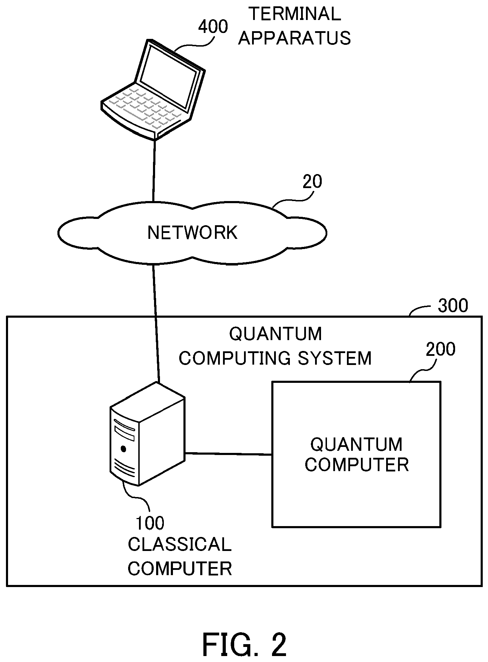

FIG. 2 is a diagram illustrating an example of a configuration of the quantum computing system. A quantum computing system 300 is a computer system using a quantum device. The quantum computing system 300 includes a classical computer 100 and a quantum computer 200. A terminal apparatus 400 is connected to the classical computer 100 via a network 20. The terminal apparatus 400 is a computer used by a user who requests the quantum computing system 300 to perform quantum computation. The classical computer 100 receives information about a many-electron system (for example, a molecule) to be solved from the terminal apparatus 400.

The classical computer 100 generates quantum circuits for computing an expected energy value of the ground state of the many-electron system based on the information about the many-electron system received from the terminal apparatus 400. The quantum circuits indicate the order of operations on qubits by the arrangement of elements such as quantum gates. The individual qubit is capable of expressing a state of superposition of a state of "0" and a state of "1". The classical computer 100 instructs the quantum computer 200 to execute quantum computation in accordance with the quantum circuits. The classical computer 100 acquires the measurement result of each qubit from the quantum computer 200.

The quantum computer 200 includes a plurality of qubits and a device for manipulating each of the plurality of qubits. The plurality of qubits included in the quantum computer 200 may be realized by, for example, a superconducting method, an ion trap method, a diamond spin method, a cold atom method (also referred to as a neutral atom method), or the like.

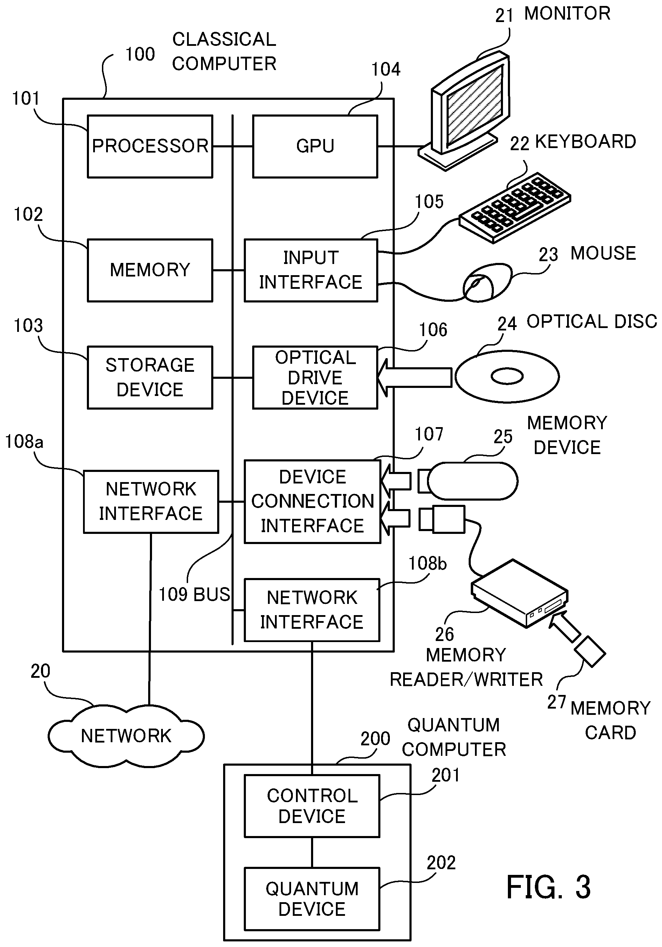

FIG. 3 is a diagram illustrating an example of hardware of apparatuses constituting the quantum computing system. The entire classical computer 100 is controlled by a processor 101. A memory 102 and a plurality of peripheral devices are connected to the processor 101 via a bus 109.

The classical computer 100 may be a multiprocessor system having a plurality of processors. A set of processors in a multiprocessor system may be referred to as a processor 101. The processor 101 may be referred to as processor circuitry. Each of the plurality of processors is able to execute some or all of the plurality of processes executed by the classical computer 100. When there are a plurality of related processes, two or more of the plurality of processes may be executed by different processors.

The processor 101 is, for example, a central processing unit (CPU), a micro processing unit (MPU), or a digital signal processor (DSP). At least a part of the functions implemented by the processor 101 executing a program may be implemented by an electronic circuit such as an application specific integrated circuit (ASIC) or a programmable logic device (PLD).

The memory 102 is used as a main storage device of the classical computer 100. The memory 102 temporarily stores at least part of an operating system (OS) program and application programs to be executed by the processor 101. The memory 102 also stores various data used for processing by the processor 101. As the memory 102, for example, a volatile semiconductor storage device such as a random access memory (RAM) is used.

Examples of the peripheral devices connected to the bus 109 include a storage device 103, a graphics processing unit (GPU) 104, an input interface 105, an optical drive device 106, a device connection interface 107, and network interfaces 108a and 108b.

The storage device 103 electrically or magnetically writes and reads data to and from a built-in recording medium. The storage device 103 is used as an auxiliary storage device of the classical computer 100. The storage device 103 stores OS programs, application programs, and various data. As the storage device 103, for example, a hard disk drive (HDD) or a solid state drive (SSD) may be used.

The GPU 104 is an arithmetic unit that performs image processing. The GPU 104 is an example of a graphic controller. A monitor 21 is connected to the GPU 104. The GPU 104 displays an image on the screen of the monitor 21 in accordance with an instruction from the processor 101. Examples of the monitor 21 include a display device using organic electro luminescence (EL) and a liquid crystal display device.

A keyboard 22 and a mouse 23 are connected to the input interface 105. The input interface 105 transmits signals sent from the keyboard 22 and the mouse 23 to the processor 101. The mouse 23 is an example of a pointing device, and other pointing devices may be used. Examples of the other pointing devices include a touch panel, a tablet, a touch pad, and a track ball.

The optical drive device 106 reads data recorded on an optical disc 24 or writes data to the optical disc 24 using laser light or the like. The optical disc 24 is a portable recording medium on which data is recorded so as to be readable by reflection of light. The optical disc 24 may be a digital versatile disc (DVD), a DVD-RAM, a compact disc read-only memory (CD-ROM), a CD-recordable (R)/ rewritable (RW), or the like.

The device connection interface 107 is a communication interface for connecting peripheral devices to the classical computer 100. For example, a memory device 25 and a memory reader/writer 26 may be connected to the device connection interface 107. The memory device 25 is a recording medium having a function of communicating with the device connection interface 107. The memory reader/writer 26 is a device that writes data to a memory card 27 or reads data from the memory card 27. The memory card 27 is a card-type recording medium.

The network interface 108a is connected to the network 20. The network interface 108a transmits and receives data to and from other computers or communication devices via the network 20. The network interface 108a is a wired communication interface connected to a wired communication device such as a switch or a router via a cable. Alternatively, the network interface 108a may be a wireless communication interface connected to a wireless communication device such as a base station or an access point by radio waves such that the network interface 108a is able to communicate with the wireless communication device.

The network interface 108b is an interface for connecting to the quantum computer 200. The processor 101 transmits quantum circuits to the quantum computer 200 via the network interface 108b, and causes the quantum computer 200 to execute quantum computation. The processor 101 acquires the result of the quantum computation via the network interface 108b.

The classical computer 100 is able to implement the processing functions according to the second embodiment by using the hardware as described above. The information processing apparatus 10 according to the first embodiment may also be implemented by hardware similar to that of the classical computer 100 illustrated in FIG. 3.

The classical computer 100 implements the processing functions according to the second embodiment by executing a program recorded in a computer-readable recording medium, for example. The program describing the processing contents to be executed by the classical computer 100 may be recorded in various recording media. For example, a program to be executed by the classical computer 100 may be stored in the storage device 103. The processor 101 loads at least a part of the programs in the storage device 103 into the memory 102 and executes the program. The program to be executed by the classical computer 100 may be recorded in a portable recording medium such as the optical disc 24, the memory device 25, or the memory card 27. The program stored in the portable recording medium becomes executable after being installed in the storage device 103 under the control of the processor 101, for example. Alternatively, the processor 101 may read the program directly from the portable recording medium and execute the program.

The quantum computer 200 includes a control device 201 and a quantum device 202. The control device 201 performs gate operations on the qubits in the quantum device 202 according to the quantum circuits. The quantum device 202 has a plurality of qubits. The quantum device 202 is, for example, one or a plurality of quantum processing units (QPUs).

With the quantum computing system 300 as described above, VQE-based quantum chemical computation is efficiently executed. Examples of the quantum chemical computation include computation of the ground state of a many-electron system.

For example, a many-electron system in which the number of electrons present is m (m is a natural number) is considered. The quantum state of the many-electron system may be expressed as follows.

The classical computer 100 first approximately solves the Schrödinger equation to obtain a possible state of one electron. Although there may be an infinite number of one-electron states obtained as solutions, the number of one-electron states herein is limited to a finite number n (n is a natural number) for convenience of numerical computation.

Due to Pauli's exclusion principle, a plurality of electrons does not take the same one electronic state. The state of the many-electron system is determined by specifying which m of the n one-electron states (for example, molecular orbitals) are occupied by electrons. Therefore, the classical computer 100 prepares n qubits that correspond to the n one-electron states and that are used for VQE on the quantum computer 200.

If the state of the i-th (i is a natural number) qubit is $|0\rangle$, this state indicates that the i-th one-electron state is not occupied by an electron. If the state of the i-th qubit is $|1\rangle$, this state indicates that the i-th one-electron state is occupied by an electron.

An approximate lowest energy state is a state in which m one-electron states having low energy among the n one-electron states are occupied by electrons. This state is called a Hartree-Fock state, and is expressed as follows on the quantum computer 200.

$$|\psi_{\texttt{Hartree-Fock}}\rangle = |1\cdots1100\cdots0\rangle$$

The numerical sequence on the right side of the Hartree-Fock state is obtained by arranging the states of the qubits corresponding to the n one-electron states from the left in ascending order of the qubit numbers. Each qubit is associated with one electron state. A qubit having a smaller qubit number has lower energy. In the Hartree-Fock state, the states of the m qubits from the smallest qubit number indicate $|1\rangle$, and the states of the other n-m qubits indicate $|0\rangle$.

The Hartree-Fock state indicates a state in which m electrons are arranged in order from one electron state having the lowest energy. In this way, in the Hartree-Fock state, since the m electrons are in a low energy state, it is considered that an approximate lowest energy state is exhibited as a whole.

On the other hand, there is a state that is not considered in the approximation by the Hartree-Fock state. Since a superposition state exists in quantum mechanics, for example, the following states also exist.

$$|\psi_1\rangle = c_0|1\cdots1100\cdots0\rangle + c_1|1\cdots1010\cdots0\rangle + c_2|1\cdots0011\cdots0\rangle$$

$c_0$, $c_1$, and $c_2$ are real number constants. In order to obtain the exact lowest energy state, the state space including all the superposition states is searched.

The quantum computing system 300 assumes the following subspace in the quantum state space of the many-electron system, and facilitates the search for the lowest energy state. First, the quantum computing system 300 defines an annihilation operator $a_i$ and a creation operator $a_i^+$ as follows.

$$a_i|\cdots1\cdots\rangle = |\cdots0\cdots\rangle, \quad a_i|\cdots0\cdots\rangle = 0$$

$$a_i^+|\cdots0\cdots\rangle = |\cdots1\cdots\rangle, \quad a_i^+|\cdots1\cdots\rangle = 0$$

$a_i$ changes a state in which the value of the i-th qubit is $|1\rangle$ to a state of $|0\rangle$. $a_i^+$ changes a state in which the value of the i-th qubit is $|0\rangle$ to a state of $|1\rangle$.

Hereinafter, the following state in which a creation operator or an annihilation operator is applied to the Hartree-Fock state will be considered.

$$|\Psi(\theta)\rangle = \exp(i\Sigma_{k,p}\theta_{k,p}a_p^+a_k+i\Sigma_{k,l,p,q}\theta_{k,l,p,q}a_q^+a_p^+a_la_k)|\psi_{\text{Hartree-Fock}}\rangle$$

$a_p^+a_k$ represents one-electron excitation that changes an electron occupying the k-th one-electron state to the p-th one-electron state. $a_q^+a_p^+a_la_k$ represents two-electron excitation that changes an electron occupying the k-th one-electron state and an electron occupying the l-th one-electron state to the p-th one-electron state and the q-th one-electron state.

A method of limiting the quantum state of the many-electron system to the quantum state represented by the above-described "$|\Psi(\theta)\rangle$" is called UCCSD ansatz. In the quantum computer 200, a qubit state corresponding to a Hartree-Fock state is first prepared, and gate operations of quantum gates corresponding to annihilation operators $_{ai}$ and creation operators $_{ai+}$ are performed on the qubit state.

In the case of the UCCSD ansatz, the number of quantum gates executed by the quantum computer 200 increases, and it is often difficult to execute quantum circuit computation. In order to execute quantum circuit computation with fewer quantum gates, various other ansatz methods have been proposed. For example, in the UCCSD ansatz, the number of electrons in the system remains unchanged even if the value of the parameter $\theta$ is changed. However, ansatz in which the number of electrons in the system is not preserved has been devised, in order to give priority to reduction in the number of quantum gates.

Therefore, in the quantum computing system 300, an equation that allows a state in which the number of electrons is not preserved is used as a function form of the trial state "$|\psi(\theta)\rangle$" with high computation efficiency. For example, the quantum computing system 300 iteratively executes computation of VQE for obtaining a state that minimizes the expected value of the equation "$\hat{H}(\mu)$" to which the penalty term including the coefficient $\mu$ is added while updating the value of $\mu$.

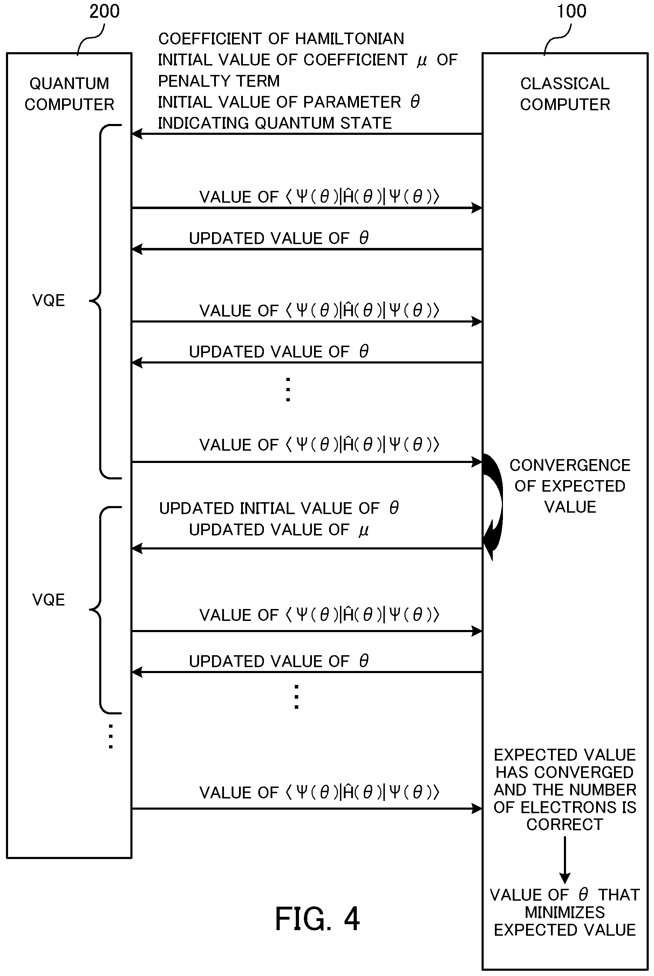

FIG. 4 is a diagram illustrating an example of a computation procedure of VQE using the equation to which the penalty term including the coefficient $\mu$ is added. For example, when the classical computer 100 acquires a computation request for VQE-based quantum computation from the terminal apparatus 400, for example, the classical computer 100 decomposes a problem to be solved into a plurality of Hamiltonians. The classical computer 100 generates a quantum circuit parameterized by the parameter $\theta$ for each decomposed Hamiltonian.

The classical computer 100 instructs the quantum computer 200 to compute the VQE using the quantum circuits obtained by the division. The instruction for the first VQE computation includes the value of the coefficient of an individual Hamiltonian, the initial value of the coefficient $\mu$ of the penalty term, the initial value of the parameter $\theta$ indicating the quantum state, and the like.

The quantum computer 200 performs quantum computation with the VQE computation quantum circuits based on the specified information. By the quantum computation, an expected value "$\langle\psi(\theta)|\hat{H}|\psi(\theta)\rangle$" of the physical quantity + the penalty term in the quantum state specified by the parameter θ is obtained. The quantum computer 200 transmits the expected value of the physical quantity + the penalty term to the classical computer 100. Then, the classical computer 100 updates the parameter θ such that the expected value of the physical quantity + the penalty term is reduced, and transmits the updated value of the parameter θ to the quantum computer 200.

In the first VQE computation, the quantum computing system 300 iteratively executes the quantum computation while updating the value of the parameter θ. When the expected value of the physical quantity + the penalty term obtained as the quantum computation result converges, the classical computer 100 ends the first VQE computation.

Each time the VQE computation ends, the classical computer 100 updates the value of the coefficient μ of the penalty term such that the number of electrons in the final state of the VQE computation becomes the correct number of electrons of the many-electron system. Then, the classical computer 100 transmits the initial value of the parameter θ and the updated value of the penalty term μ in the next VQE computation to the quantum computer 200, and starts the next VQE computation. The classical computer 100 sets the value of the parameter θ indicating the state of each qubit in the previous VQE computation as the initial value of the parameter θ of the next VQE computation.

In this way, the classical computer 100 and the quantum computer 200 iteratively execute the VQE computation while updating the value of the coefficient μ. When the number of electrons obtained when the expected value of the physical quantity + the penalty term computed by the VQE converges reaches the correct number, the classical computer 100 ends the iteration of the VQE computation. The classical computer 100 outputs, as a computation result, the value of the parameter θ that minimizes the expected value of the physical quantity + the penalty term, the value of the parameter θ having been obtained when the iteration of the VQE computation ends.

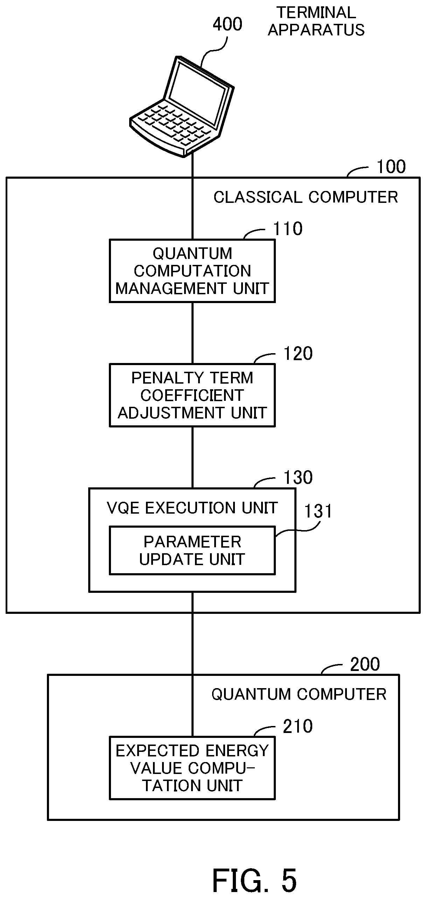

FIG. 5 is a block diagram illustrating an example of functions of the classical computer that performs quantum chemical computation. The classical computer 100 includes a quantum computation management unit 110, a penalty term coefficient adjustment unit 120, and a VQE execution unit 130, in order to execute quantum chemical computation.

The quantum computation management unit 110 receives a VQE computation request from, for example, the terminal apparatus 400. The quantum computation management unit 110 transmits, to the penalty term coefficient adjustment unit 120, information about the many-electron system to be solved indicated in the computation request. In addition, the quantum computation management unit 110 acquires the result of the VQE from the penalty term coefficient adjustment unit 120, and transmits the computation result to the terminal apparatus 400.

The penalty term coefficient adjustment unit 120 generates a Hamiltonian equation including a penalty term based on the information about the many-electron system to be solved, and determines the coefficient of the penalty term. Then, the penalty term coefficient adjustment unit 120 generates quantum circuits for computing a solution of the generated equation based on the VQE, and transmits the generated quantum circuits to the VQE execution unit 130.

In addition, when the penalty term coefficient adjustment unit 120 acquires the VQE computation result from the VQE execution unit 130, the penalty term coefficient adjustment unit 120 determines whether the number of electrons indicated in the computation result is correct. If the number of electrons is correct, the penalty term coefficient adjustment unit 120 determines that the quantum chemical computation is completed. When the quantum chemical computation ends, the penalty term coefficient adjustment unit 120 transmits the expected energy value to the quantum computation management unit 110 as a computation result. If there is an error in the number of electrons, the penalty term coefficient adjustment unit 120 updates the coefficient of the penalty term and instructs the VQE execution unit 130 to perform the VQE computation to which the updated coefficient has been applied.

Upon acquiring the quantum circuits for the VQE computation, the VQE execution unit 130 executes the VQE based on the acquired quantum circuits in cooperation with the quantum computer 200. When the value of the coefficient of the penalty term is updated, the VQE execution unit 130 cooperates with the quantum computer 200 to execute the VQE to which the updated coefficient has been applied. In order to execute the VQE, the VQE execution unit 130 includes a parameter update unit 131 that optimizes the parameter θ used for the VQE.

For example, the VQE execution unit 130 first specifies the initial value of the parameter θ and instructs the quantum computer 200 to execute the quantum circuits with the parameter θ. The VQE execution unit 130 obtains an expected energy value as the execution result of the quantum circuits. Upon acquiring the expected energy value, the parameter update unit 131 updates the parameter θ such that the expected energy value is reduced. The VQE execution unit 130 instructs the quantum computer 200 to execute the quantum circuits with the updated parameter θ.

The VQE execution unit 130 iterates the update of the parameter θ and the execution of the quantum circuits using the updated parameter θ until the VQE computation result (for example, the energy expected value) converges. When the expected energy value converges, the VQE execution unit 130 transmits the value of the parameter θ indicating the state at that time to the penalty term coefficient adjustment unit 120 as the VQE computation result.

The quantum computer 200 includes an expected energy value computation unit 210. The expected energy value computation unit 210 executes gate operations on the qubits based on the quantum circuits entered from the classical computer 100, and measures a final qubit state. Then, the expected energy value computation unit 210 computes an expected value of the physical quantity + the penalty term from the measurement result, and transmits the expected value to the classical computer 100. The process of computing the expected value of the physical quantity + the penalty term from the measurement result may be executed by the VQE execution unit 130 of the classical computer 100.

The functions of the elements in the classical computer 100 illustrated in FIG. 5 may be implemented by causing the processor 101 to execute program modules corresponding to the elements, for example.

Next, a method of updating the coefficient of the penalty term will be described. The Hamiltonian (physical quantity + penalty term) Ĥ(μ) to which the penalty term has been added is expressed by the following equation (1).

$$\widehat{H}(\mu) = \sum_{p,q=1}^{N} h_{pq} a_p^+ a_q + \sum_{p,q,r,s=1}^{N} h_{pqrs} a_p^+ a_q^+ a_r a_s - \mu \sum_{p=1}^{N} a_p^+ a_p \quad (1)$$

The third term (last term) on the right side of Equation (1) is a penalty term. The penalty term is obtained by multiplying an operator ($\Sigma_p = 1^N a_p^+ a_p$) for acquiring the number of electrons by the coefficient μ.

The penalty term coefficient adjustment unit 120 adjusts the coefficient μ of the penalty term by using, for example, a function for obtaining an error in the number of electrons.

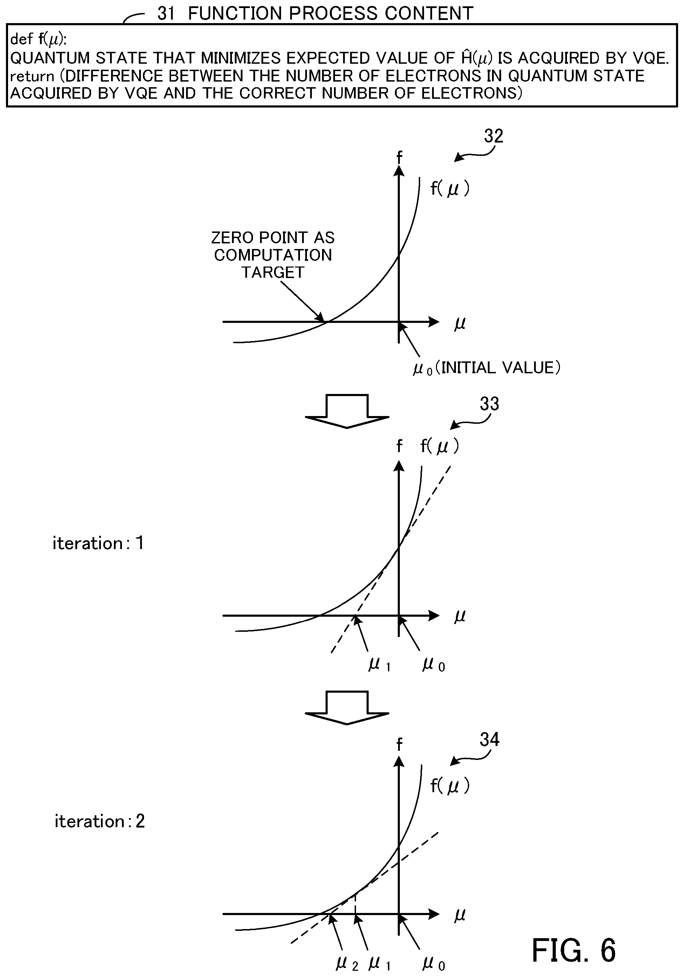

FIG. 6 is a diagram illustrating an example of a method of adjusting the coefficient μ of the penalty term. In order to adjust the coefficient μ of the penalty term, the penalty term coefficient adjustment unit 120 defines a function f(μ) that executes the process indicated in the function process content 31. The process executed based on the function f(μ) is as follows.

[Process 1] A process of acquiring a quantum state that minimizes the expected value of Ĥ(μ) by VQE.
[Process 2] A process of outputting the difference between the number of electrons in the quantum state acquired by the VQE and the correct number of electrons as a return value (return).

The penalty term coefficient adjustment unit 120 uses the return value (return) of the function f(μ), to search for μ that satisfies f(μ) = 0 in accordance with the Newton method, for example.

Graphs 32 to 34 illustrate examples of updating the value of μ based on the function f(μ). The horizontal axis of the graphs 32 to 34 represents the value of the coefficient μ, and the vertical axis represents a value f obtained by the function f(μ).

For example, the penalty term coefficient adjustment unit 120 first sets an initial value $\mu_0$ to the coefficient μ and executes the function f(μ). In the first iteration (iteration: 1), the penalty term coefficient adjustment unit 120 obtains an intersection point between a tangent line that is tangent to the curve indicating the function f(μ) at a position of $\mu = \mu_0$ and a line of f = 0. The penalty term coefficient adjustment unit 120 sets the value of μ at the obtained intersection as the updated value $\mu_1$ of the coefficient μ.

Next, the penalty term coefficient adjustment unit 120 sets the updated value $\mu_1$ to the coefficient μ and executes the function f(μ). In the second iteration (iteration: 2), the penalty term coefficient adjustment unit 120 obtains an intersection point between a tangent line that is tangent to the curve indicating the function f(μ) at the position of $\mu = \mu_1$ and the line of f = 0. The penalty term coefficient adjustment unit 120 sets the value of μ at the obtained intersection as the updated coefficient value $\mu_2$.

In this way, the penalty term coefficient adjustment unit 120 updates the value of the coefficient μ such that the value of the coefficient μ approaches a value at which the value obtained by the function f(μ) becomes "0" (no error). Finally, an appropriate value of the coefficient μ of the penalty term is obtained.

Next, a procedure of a VQE-based quantum chemical computation process will be described in detail.

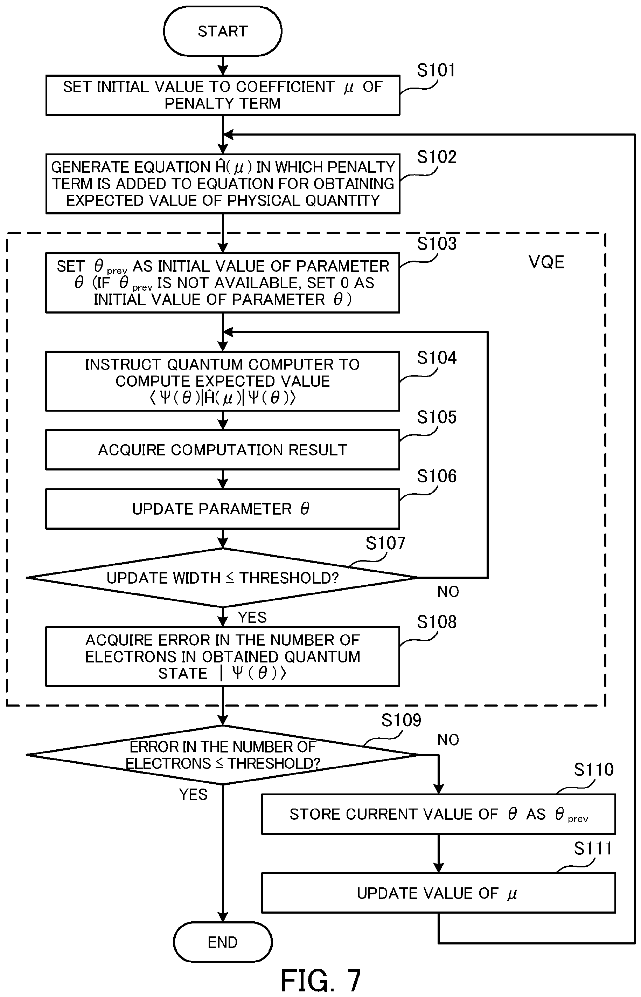

FIG. 7 is a flowchart illustrating an example of a procedure of a VQE-based quantum chemical computation process. Hereinafter, the process illustrated in FIG. 7 will be described in order of step numbers.

[Step S101] The penalty term coefficient adjustment unit 120 sets an initial value to the coefficient μ of the penalty term.

[Step S102] The penalty term coefficient adjustment unit 120 generates an equation "Ĥ(μ)" in which a penalty term is added to an equation for obtaining an expected value of a physical quantity (for example, energy). Then, the penalty term coefficient adjustment unit 120 executes the function f(μ) illustrated in FIG. 6. When the execution of the function f(μ) is started, the penalty term coefficient adjustment unit 120 first instructs the VQE execution unit 130 to perform VQE computation based on "Ĥ(μ)". The VQE execution unit 130 performs VQE computation for obtaining a quantum state "|Ψ(θ)⟩" that minimizes "⟨Ψ(θ)|Ĥ(μ)|Ψ(θ)⟩". The procedure of the VQE computation process is executed in accordance with the following steps S103 to S108.

[Step S103] The VQE execution unit 130 sets an initial value of the parameter θ in the trial state "|Ψ(θ)⟩". For example, if $\theta_{prev}$ is not available, the VQE execution unit 130 sets the initial value of θ to 0. This state corresponds to the Hartree-Hock state. When $\theta_{prev}$ is available, the VQE execution unit 130 sets the value of $\theta_{prev}$ as the initial value of θ.

[Step S104] The VQE execution unit 130 instructs the quantum computer 200 to compute the expected value "$\langle\Psi(\theta)|\hat{H}(\mu)|\Psi(\theta)\rangle$" of the physical quantity + the penalty term in the trial state. The quantum computer 200 obtains the expected value "$\langle\Psi(\theta)|\hat{H}(\mu)|\Psi(\theta)\rangle$" of the physical quantity + the penalty term by executing quantum computation in accordance with the instruction. A specific method of computing "$\langle\Psi(\theta)|\hat{H}(\mu)|\Psi(\theta)\rangle$" will be described below.

In a many-electron system, if it is known which one-electron states are occupied by the electrons, operators for obtaining the energy of the state may be expressed as follows (second quantization Hamiltonian).

$$\hat{H} = \sum_{i,j} h_{i,j} a_i^+ a_j + \sum_{i,j,k,l} h_{i,j,k,l} a_i^+ a_j^+ a_k a_l \qquad (2)$$

$a_i^+$ and $a_j^+$ on the right side are creation operators in a one-electron state. $a_j$, $a_k$, and $a_l$ are annihilation operators in one electron state. $h_{i,j}$ and $h_{i,j,k,l}$ are real numbers. The first term on the right side is a one-electron Hamiltonian and is a term derived from the kinetic energy of an electron and the Coulomb attractive energy between an electron and an atomic nucleus. The second term on the right side is a two-electron Hamiltonian and is a term derived from the Coulomb repulsion energy between two electrons or the like. The process of applying the operator of each term to the state "$|\Psi(\theta)\rangle$" may be realized by applying quantum gates to qubits on the quantum computer 200.

An equation obtained by rewriting the second quantization Hamiltonian in a language corresponding to the quantum gates is referred to as a qubit Hamiltonian. The quantum computer 200 computes an expected value "$\langle\Psi(\theta)|\hat{H}(\mu)|\Psi(\theta)\rangle$" of the physical quantity of the state + the penalty term by executing quantum circuits corresponding to the qubit Hamiltonians.

[Step S105] The VQE execution unit 130 acquires the computation result of the expected value "$\langle\Psi(\theta)|\hat{H}(\mu)|\Psi(\theta)\rangle$" of the physical quantity + and the penalty term in the trial state from the quantum computer 200.

[Step S106] The parameter update unit 131 of the VQE execution unit 130 updates the value of the parameter $\theta$ in order to minimize the expected value of the physical quantity + the penalty term. Here, the expected value of the physical quantity + the penalty term "$E(\theta) = \langle\Psi(\theta)|\hat{H}(\mu)|\Psi(\theta)\rangle$" is a continuous function related to the parameter $\theta$. As a method of updating the parameter $\theta$ for minimizing the objective function, for example, a gradient descent method is used. The gradient descent method is represented by the following equation.

$$\boldsymbol{\theta} \rightarrow \boldsymbol{\theta} - \eta \frac{\partial E(\boldsymbol{\theta})}{\partial \boldsymbol{\theta}} \qquad (3)$$

$\eta$ is a real number. The differential coefficient "$\partial E(\theta)/\partial\theta$" is obtained by, for example, numerical differentiation. In this case, the VQE execution unit 130 causes the quantum computer 200 to compute not only the value of $E(\theta)$ but also the value of "$E(\theta + \delta)$" when causing the quantum computer 200 to execute quantum computation. The parameter update unit 131 computes the value of $\theta$ updated according to Equation (3) using the acquired $E(\theta)$ and $E(\theta + \delta)$.

[Step S107] The VQE execution unit 130 determines whether the update width of the parameter $\theta$ (the difference between the values of the parameter $\theta$ before and after the update) is equal to or less than a threshold. That is, when the value of the parameter $\theta$ converges and the update width becomes equal to or less than the threshold, it is considered that the expected value of the physical quantity + the penalty term has also converged. If the update width is equal to or less than the threshold, the VQE execution unit 130 advances the process to step S108. If the update width exceeds the threshold, the VQE execution unit 130 advances the process to step S104.

[Step S108] The VQE execution unit 130 acquires an error in the expected value "$\langle\Psi(\theta)|\hat{N}|\Psi(\theta)\rangle$" (N is with ^) of the number of electrons in the obtained quantum state "$|\Psi(\theta)\rangle$" from the correct number of electrons. For example, the number of electrons in a state in a many-electron system may be obtained by counting the number of one-electron states occupied by electrons. Therefore, the operators for obtaining the number of electrons may be expressed as follows.

$$\hat{N} = \sum_i a_i^+ a_i \qquad (4)$$

$a_i^+$ and $a_i$ on the right side of Equation (4) are a creation operator and an annihilation operator in a one-electron state, respectively. The expected value "$\langle\Psi(\theta)|\hat{N}|\Psi(\theta)\rangle$" (N is with ^) of the number of electrons in the state is also obtained as a result of the quantum circuit computation on the quantum computer 200.

The VQE execution unit 130 transmits the acquired error in the number of electrons to the penalty term coefficient adjustment unit 120.

[Step S109] The penalty term coefficient adjustment unit 120 determines whether the error in the number of electrons is equal to or less than a threshold. When the ground state in which the correct number of electrons is obtained is finally obtained, the threshold of the error in the number of electrons is "0". If the error in the number of electrons is equal to or less than the threshold, the penalty term coefficient adjustment unit 120 transmits the computation result such as the expected value of the physical quantity (for example, energy) at that time and the value of the parameter $\theta$ at that time to the quantum computation management unit 110, and ends the process. If the error in the number of electrons exceeds the threshold, the penalty term coefficient adjustment unit 120 advances the process to step S110.

[Step S110] The penalty term coefficient adjustment unit 120 stores the current value of $\theta$ as $\theta_{prev}$ in the memory 102 or the storage device 103.

[Step S111] The penalty term coefficient adjustment unit 120 updates the value of the coefficient $\mu$ of the penalty term. Thereafter, the penalty term coefficient adjustment unit 120 advances the process to step S102.

In this way, the quantum computing system 300 is able to efficiently compute the expected value of the physical quantity + the penalty term by executing the VQE computation in which the number of electrons is not preserved. At this time, by iterating the VQE computation until the error in the number of electrons becomes equal to or less than the threshold, the expected value of the physical quantity + the penalty term, the expected value corresponding to the correct number of electrons, is finally obtained. In addition, in the second and subsequent VQE computations, the value of the parameter $\theta$ obtained in the previous VQE computation is set as the initial value of the parameter $\theta$ in the next VQE computation. As a result, the expected value of the physical quantity + the penalty term in the VQE computation is allowed to converge with a small number of iterations, and the computation time per VQE computation is shortened.

[Third Embodiment]

In a third embodiment, a VQE computation closest to the value of the coefficient $\mu$ set in the k-th VQE computation is selected from the first to (k - 1)th VQE computations, and the state obtained as a result of the selected VQE computation is set as the initial value of the k-th VQE computation. This method of updating the coefficient $\mu$ is effective when the conversion of $\mu$ by the Newton method is not monotonic (monotonic increase or monotonic decrease).

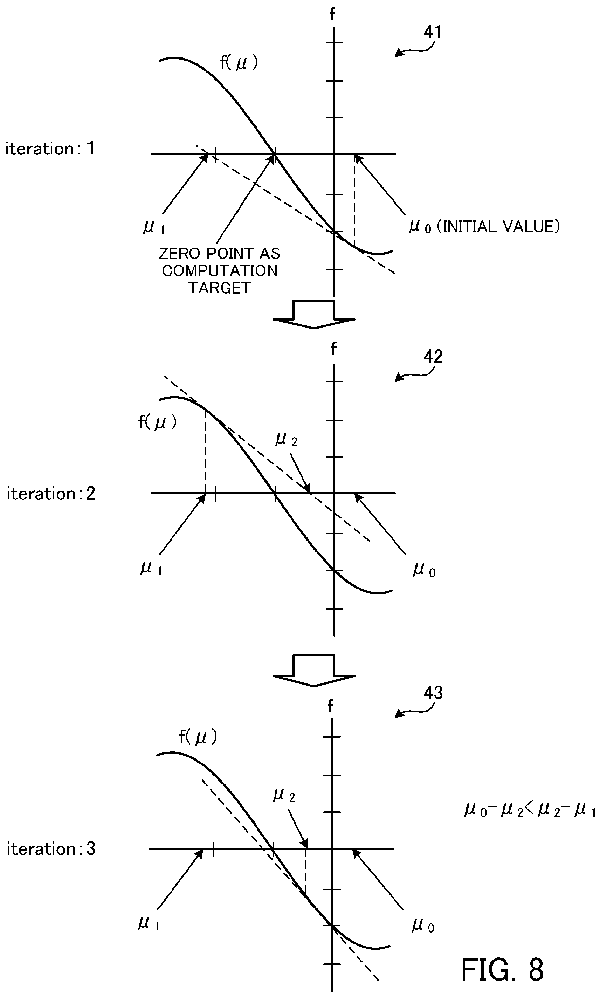

FIG. 8 is a diagram illustrating an example of a case where the conversion of the coefficient $\mu$ by the Newton method is not monotonic. The case where the change of the coefficient $\mu$ is monotonic is, for example, a case where the value of $\mu$ decreases as the number of executions of the VQE computation (subscript of $\mu$) increases, as expressed by "$\mu_0 > \mu_1 > \mu_2 > \cdots$". Conversely, when the value of $\mu$ increases as the number of executions of the VQE computation increases, the change is also considered to be monotonic.

Graphs 41 to 43 illustrate an update example of the value of the coefficient $\mu$ based on the function $f(\mu)$ in a case where the value of the coefficient $\mu$ does not monotonically change. In the graphs 41 to 43, the horizontal axis represents the value of the coefficient $\mu$, and the vertical axis represents a value f obtained by the function $f(\mu)$.

For example, the penalty term coefficient adjustment unit 120 first sets an initial value $\mu_0$ to the coefficient $\mu$ and executes the function $f(\mu)$. By executing the function $f(\mu)$, the VQE computation is performed, and the minimum value of the expected value of the physical quantity + the penalty term in the first iteration (iteration: 1) is obtained. The value of the parameter $\theta$ obtained when the minimum value of the expected value of the physical quantity + the penalty term at this time is obtained is expressed as follows.

$$\operatorname*{argmin}_{\theta} \langle \Psi(\theta)|\widehat{H}(\mu_0)|\Psi(\theta)\rangle \qquad (5)$$

In the first iteration, the penalty term coefficient adjustment unit 120 obtains an intersection point between a tangent line that is tangent to the curve indicating the function $f(\mu)$ at a position of $\mu = \mu_0$ and a line of $f = 0$. The penalty term coefficient adjustment unit 120 sets the value of $\mu$ at the obtained intersection as the updated value $\mu_1$ of the coefficient $\mu$.

Next, the penalty term coefficient adjustment unit 120 sets the updated value $\mu_1$ to the coefficient $\mu$ and executes the function $f(\mu)$. By executing the function $f(\mu)$, the VQE computation is performed by the VQE execution unit 130, and the minimum value of the expected value of the physical quantity + the penalty term in the second iteration (iteration: 2) is obtained. The value of the parameter $\theta$ obtained when the minimum value of the expected value of the physical quantity + the penalty term is obtained is expressed as follows.

$$\operatorname*{argmin}_{\theta} \langle \Psi(\theta)|\widehat{H}(\mu_1)|\Psi(\theta)\rangle \qquad (6)$$

In the second iteration, the penalty term coefficient adjustment unit 120 obtains an intersection point between a tangent line that is tangent to the curve indicating the function $f(\mu)$ at the position of $\mu = \mu_1$ and the line of $f = 0$. The penalty term coefficient adjustment unit 120 sets the value of $\mu$ at the obtained intersection as the updated coefficient value $\mu_2$.

In the example of FIG. 8, "$\mu_0 > \mu_2 > \mu_1$", and the change in the value of the coefficient $\mu$ is not monotonic. The value of $\mu_2$ is closer to $\mu_0$ than to $\mu_1$ ($\mu_0 - \mu_2 < \mu_2 - \mu_1$). In this case, it is considered that using the value of Equation (5) obtained in the first iteration as the initial value of the parameter $\theta$ in the third iteration (iteration: 3) achieves higher VQE computation efficiency than using the value of Equation (6) obtained in the second iteration.

Therefore, in the example of FIG. 8, the penalty term coefficient adjustment unit 120 sets the value of the parameter $\theta$ obtained when the minimum value of the expected value of the physical quantity + the penalty term in the first iteration is obtained as the initial value of the parameter $\theta$ of the VQE computation in the third iteration.

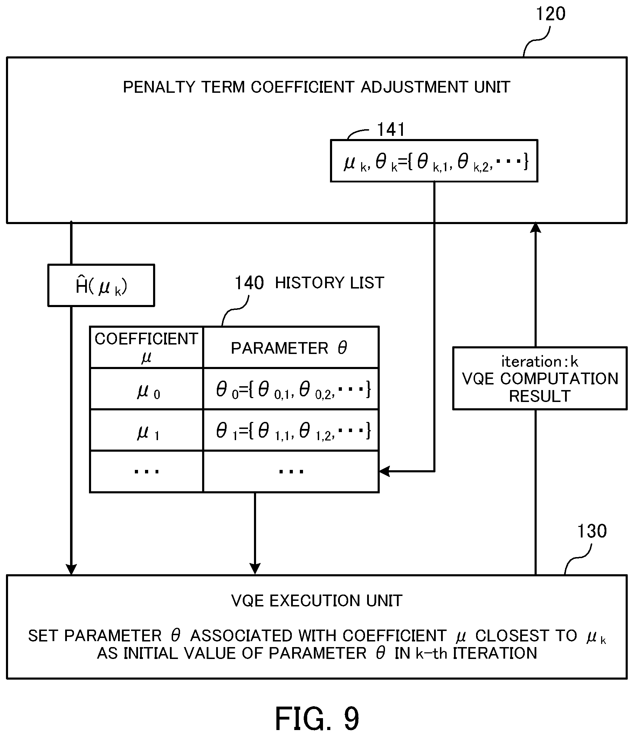

FIG. 9 is a diagram illustrating an example of a method of determining the initial value of the parameter $\theta$ of the next VQE computation from the computation results of a plurality of past VQEs. For example, a history list 140 is provided in the memory 102 or the storage device 103 of the classical computer 100. In the history list 140, in association with the value of the coefficient $\mu$ set in the individual VQE computation executed, the value of the parameter $\theta$ obtained when the expected value of the physical quantity + the penalty term becomes the minimum in the corresponding VQE computation is set. The individual parameter $\theta$ is, for example, data including a plurality of parameter values indicating rotation angles of rotation operations by a plurality of rotation gates included in the quantum circuits used for the corresponding VQE computation.

The penalty term coefficient adjustment unit 120 determines a value $\mu_k$ of the coefficient $\mu$ to be applied to the k-th VQE computation. Then, the penalty term coefficient adjustment unit 120 transmits an equation $H(\mu_k)$ obtained by adding the penalty term to the equation for obtaining the expected value of the physical quantity to the VQE execution unit 130.

The VQE execution unit 130 searches for a value closest to $\mu_k$ from the values of the coefficients $\mu$ set in the history list 140. The VQE execution unit 130 extracts the value of the parameter $\theta$ corresponding to the value of the matching coefficient $\mu$ from the history list 140. The VQE execution unit 130 sets the extracted value of the parameter $\theta$ to the initial value of the parameter $\theta$ in the VQE computation, and executes the VQE computation. Then, the VQE execution unit 130 transmits the computation result of the k-th VQE to the penalty term coefficient adjustment unit 120. The penalty term coefficient adjustment unit 120 adds a set of the value $\mu_k$ of the coefficient $\mu$ applied to the k-th VQE computation and the value $\theta_k$ of the parameter $\theta$ obtained when the expected value of the physical quantity + the penalty term is minimized in the k-th VQE computation to the history list 140 as one record 141.

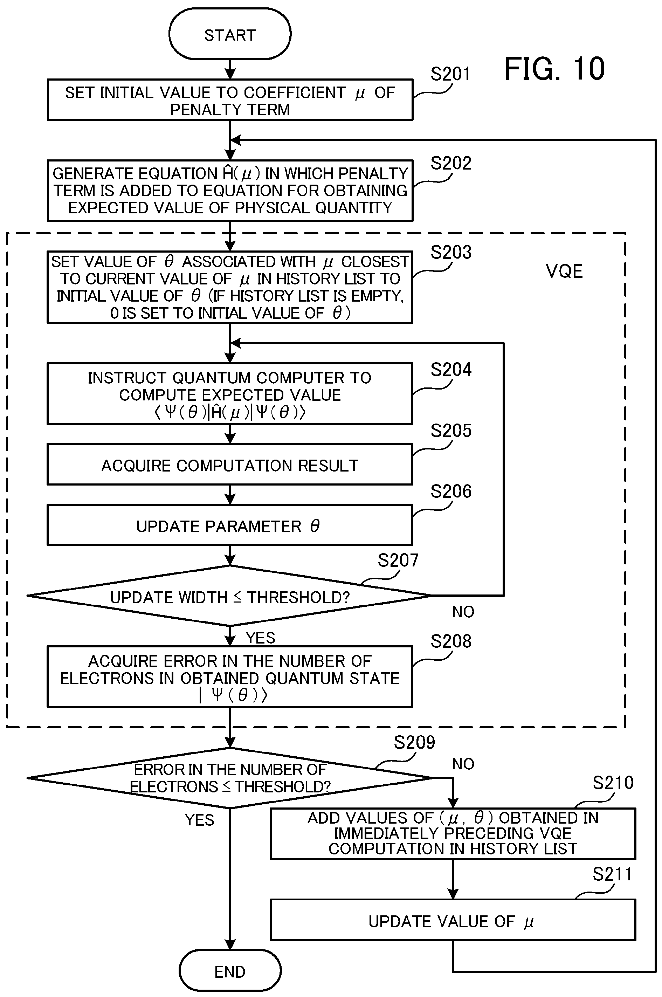

FIG. 10 is a flowchart illustrating an example of a procedure of a VQE-based quantum chemical computation process according to the third embodiment. In the process illustrated in FIG. 10, steps S201 and S202, S204 to S209, and S211 are the same as steps S101 and S102, S104 to S109, and S111 (see FIG. 7) according to the second embodiment, respectively. Steps S203 and S210 different from those according to the second embodiment are as follows.

[Step S203] The VQE execution unit 130 refers to the history list 140 generated in the following step S210, and sets the initial value of the parameter $\theta$ in the trial state "$|\Psi(\theta)\rangle$". In the first VQE iteration, the history list 140 is empty. In this case, for example, the VQE execution unit 130 sets the initial value of the parameter $\theta$ to "0" (Hartree-Fock state).

If the history list is not empty, the VQE execution unit 130 specifies a record ($\mu$, $\theta$) having $\mu$ closest to the current value of $\mu$ in the history list. Then, the VQE execution unit 130 sets the value of $\theta$ set in the specified record as the initial value of the parameter $\theta$ in the current VQE computation.

Thereafter, the VQE execution unit 130 and the quantum computer 200 cooperate with each other to execute the VQE computation. When the expected value of the physical quantity + the penalty term obtained by the VQE computation converges to the minimum value, the computation result is transmitted from the VQE execution unit 130 to the penalty term coefficient adjustment unit 120. If an error in the number of electrons in the state indicated by the computation result exceeds a threshold, step S210 is performed by the penalty term coefficient adjustment unit 120.

[Step S210] The penalty term coefficient adjustment unit 120 adds, to the history list 140, a record including the value of the coefficient $\mu$ applied to the immediately preceding VQE computation and the value of the parameter $\theta$ obtained when the expected value of the physical quantity + the penalty term obtained by that VQE computation becomes the minimum value.

Thereafter, the value of the coefficient $\mu$ is updated by the penalty term coefficient adjustment unit 120, and the VQE computation to which the updated value of the coefficient $\mu$ has been applied is performed.

In this way, even if the value of the coefficient $\mu$ does not monotonically increase or monotonically decrease, an appropriate value is set as the initial value of the parameter $\theta$ for each VQE computation.

Although the embodiments have been described above as examples, the configuration of a unit described in the embodiments may be replaced with another configuration having an equivalent function. Any other components or steps may be added. Furthermore, any two or more configurations (features) of the above-described embodiments may be combined.

In one aspect, the efficiency of VQE computation is improved.

**Claims**

1. A computer program that causes a computer to execute a process comprising:

   alternately iterating a process of updating a value of a coefficient in a third equation obtained by adding a term obtained by multiplying a second equation relating to a number of electrons in a many-electron system by the coefficient to a first equation for computing a physical quantity of the many-electron system, and a search process of searching for a ground state of the many-electron system based on a variational quantum eigensolver method by changing a quantum state of the many-electron system such that an expected value of the third equation based on the quantum state of the many-electron system is reduced,
   wherein the search process in a (k + 1)th iteration, wherein k is a natural number, includes setting of a final state of the many-electron system obtained in the search process in any one of first to k-th iterations as an initial state of the many-electron system in the search process in the (k + 1)th iteration, and changing of the quantum state of the many-electron system from the initial state such that the expected value of the third equation is reduced.

2. The computer program according to claim 1, wherein the search process in the (k + 1)th iteration includes setting of the final state of the many-electron system in the search process in the k-th iteration as the initial state of the many-electron system in the search process in the (k + 1)th iteration.

3. The computer program according to claim 1 or claim 2, wherein the search process in the (k + 1)th iteration includes selecting one value based on a difference between an individual value of the coefficient of the third equation used in the search process in the first to k-th iterations and the value of the coefficient of the third equation used in the search process in the (k + 1)th iteration, and setting of the final state of the many-electron system in the search process executed using the third equation in which the selected one value is set as the value of the coefficient as the initial state of the many-electron system in the search process in the (k + 1)th iteration.

4. The computer program according to any preceding claim, wherein the search process includes changing the quantum state of the many-electron system while allowing fluctuation in the number of electrons in the many-electron system, and determining, in response to the number of electrons in the many-electron system in the final state of the many-electron system satisfying a predetermined condition, the final state of the many-electron system as the ground state of the many-electron system.

5. A quantum chemical computation method executed by a computer, the quantum chemical computation method comprising:

   alternately iterating a process of updating a value of a coefficient in a third equation obtained by adding a term obtained by multiplying a second equation relating to a number of electrons in a many-electron system by the coefficient to a first equation for computing a physical quantity of the many-electron system, and a search process of searching for a ground state of the many-electron system based on a variational quantum eigensolver method by changing a quantum state of the many-electron system such that an expected value of the third equation based on the quantum state of the many-electron system is reduced; and
   setting in the search process in a (k + 1)th iteration, wherein k is a natural number, a final state of the many-electron system obtained in the search process in any one of first to k-th iterations as an initial state of the many-electron system in the search process in the (k + 1)th iteration, and changing, by the processor, the quantum state of the many-electron system from the initial state such that the expected value of the third equation is reduced.

6. An information processing apparatus comprising:
   processing means for:

   alternately iterating a process of updating a value of a coefficient in a third equation obtained by adding a term obtained by multiplying a second equation relating to a number of electrons in a many-electron system by the coefficient to a first equation for computing a physical quantity of the many-electron system, and a search process of searching for a ground state of the many-electron system based on a variational quantum eigensolver method by changing a quantum state of the many-electron system such that an expected value of the third equation based on the quantum state of the many-electron system is reduced,
   wherein the search process in a (k + 1)th iteration, wherein k is a natural number, includes setting of a final state of the many-electron system obtained in the search process in any one of first to k-th iterations as an initial state of the many-electron system in the search process in the (k + 1)th iteration, and changing of the quantum state of the many-electron system from the initial state such that the expected value of the third equation is reduced.

THIRD
EQUATION

FIRST
EQUATION

SECOND
EQUATION

QUANTUM STATE OF
MANY–ELECTRON SYSTEM
$|\Psi(\theta)\rangle$

$$\hat{H}(\mu):=H+\mu\,C$$

COEFFICIENT

PENALTY TERM

SET COEFFICIENT $\mu$ : $\mu = \mu_1$

1st VQE          INITIAL VALUE OF $\theta = 0$

EXPECTED VALUE: $\langle \Psi(\theta)|\hat{H}(\mu_1)|\Psi(\theta)\rangle$

OPTIMIZE $\theta$

FINAL STATE (CONVERGENCE OF
EXPECTED VALUE): $|\Psi(\theta_1)\rangle$

UPDATE COEFFICIENT $\mu$ : $\mu = \mu_2$

$\vdots$

kth VQE          INITIAL VALUE OF $\theta = \theta_{k-1}$

EXPECTED VALUE: $\langle \Psi(\theta)|\hat{H}(\mu_k)|\Psi(\theta)\rangle$

OPTIMIZE $\theta$

FINAL STATE (CONVERGENCE OF
EXPECTED VALUE): $|\Psi(\theta_k)\rangle$

UPDATE COEFFICIENT $\mu$ : $\mu = \mu_{k+1}$

(k + 1)th VQE    INITIAL VALUE OF $\theta = \theta_k$

EXPECTED VALUE: $\langle \Psi(\theta)|\hat{H}(\mu_{k+1})|\Psi(\theta)\rangle$

OPTIMIZE $\theta$

FINAL STATE (CONVERGENCE OF
EXPECTED VALUE): $|\Psi(\theta_{k+1})\rangle$

$\vdots$

INFORMATION PROCESSING
APPARATUS          11

10

STORAGE
UNIT

PROCESSING
UNIT          12

1

QUANTUM
COMPUTER

FIG. 1

TERMINAL
APPARATUS
400

20
NETWORK

300
QUANTUM
COMPUTING SYSTEM
200

QUANTUM
COMPUTER

100
CLASSICAL
COMPUTER

FIG. 2

**FIG. 3**

200                     100

| QUANTUM COMPUTER | | CLASSICAL COMPUTER |
|---|---|---|

COEFFICIENT OF HAMILTONIAN
INITIAL VALUE OF COEFFICIENT $\mu$ OF PENALTY TERM
INITIAL VALUE OF PARAMETER $\theta$ INDICATING QUANTUM STATE

VQE

VALUE OF $\langle \Psi(\theta)|\hat{H}(\theta)|\Psi(\theta)\rangle$

UPDATED VALUE OF $\theta$

VALUE OF $\langle \Psi(\theta)|\hat{H}(\theta)|\Psi(\theta)\rangle$

UPDATED VALUE OF $\theta$

⋮

VALUE OF $\langle \Psi(\theta)|\hat{H}(\theta)|\Psi(\theta)\rangle$

CONVERGENCE OF EXPECTED VALUE

UPDATED INITIAL VALUE OF $\theta$
UPDATED VALUE OF $\mu$

VQE

VALUE OF $\langle \Psi(\theta)|\hat{H}(\theta)|\Psi(\theta)\rangle$

UPDATED VALUE OF $\theta$

⋮

⋮

VALUE OF $\langle \Psi(\theta)|\hat{H}(\theta)|\Psi(\theta)\rangle$

EXPECTED VALUE HAS CONVERGED AND THE NUMBER OF ELECTRONS IS CORRECT

VALUE OF $\theta$ THAT MINIMIZES EXPECTED VALUE

# FIG. 4

TERMINAL
APPARATUS

400

100

CLASSICAL COMPUTER

110

QUANTUM
COMPUTATION
MANAGEMENT UNIT

120

PENALTY TERM
COEFFICIENT
ADJUSTMENT UNIT

130

VQE EXECUTION UNIT

131

PARAMETER
UPDATE UNIT

200

QUANTUM COMPUTER

210

EXPECTED ENERGY
VALUE COMPU-
TATION UNIT

# FIG. 5

## 31 FUNCTION PROCESS CONTENT

```
def f(μ):
QUANTUM STATE THAT MINIMIZES EXPECTED VALUE OF Ĥ(μ) IS ACQUIRED BY VQE.
return (DIFFERENCE BETWEEN THE NUMBER OF ELECTRONS IN QUANTUM STATE
ACQUIRED BY VQE AND THE CORRECT NUMBER OF ELECTRONS)
```

32

$f$

$f(\mu)$

ZERO POINT AS
COMPUTATION
TARGET

$\mu$

$\mu_0$(INITIAL VALUE)

33

$f$  $f(\mu)$

iteration : 1

$\mu$

$\mu_1$    $\mu_0$

34

$f$

$f(\mu)$

iteration : 2

$\mu$

$\mu_2$ $\mu_1$    $\mu_0$

FIG. 6

START

SET INITIAL VALUE TO COEFFICIENT $\mu$ OF PENALTY TERM — S101

GENERATE EQUATION $\hat{H}(\mu)$ IN WHICH PENALTY TERM IS ADDED TO EQUATION FOR OBTAINING EXPECTED VALUE OF PHYSICAL QUANTITY — S102

VQE

SET $\theta_{prev}$ AS INITIAL VALUE OF PARAMETER $\theta$ (IF $\theta_{prev}$ IS NOT AVAILABLE, SET 0 AS INITIAL VALUE OF PARAMETER $\theta$) — S103

INSTRUCT QUANTUM COMPUTER TO COMPUTE EXPECTED VALUE $\langle \Psi(\theta)|\hat{H}(\mu)|\Psi(\theta)\rangle$ — S104

ACQUIRE COMPUTATION RESULT — S105

UPDATE PARAMETER $\theta$ — S106

UPDATE WIDTH ≤ THRESHOLD? — S107

NO

YES

ACQUIRE ERROR IN THE NUMBER OF ELECTRONS IN OBTAINED QUANTUM STATE $|\Psi(\theta)\rangle$ — S108

ERROR IN THE NUMBER OF ELECTRONS ≤ THRESHOLD? — S109

NO

YES

STORE CURRENT VALUE OF $\theta$ AS $\theta_{prev}$ — S110

UPDATE VALUE OF $\mu$ — S111

END

FIG. 7

EP 4 651 039 A1

FIG. 8

FIG. 9

# FIG. 10

START

SET INITIAL VALUE TO COEFFICIENT $\mu$ OF PENALTY TERM — S201

GENERATE EQUATION $\hat{H}(\mu)$ IN WHICH PENALTY TERM IS ADDED TO EQUATION FOR OBTAINING EXPECTED VALUE OF PHYSICAL QUANTITY — S202

SET VALUE OF $\theta$ ASSOCIATED WITH $\mu$ CLOSEST TO CURRENT VALUE OF $\mu$ IN HISTORY LIST TO INITIAL VALUE OF $\theta$ (IF HISTORY LIST IS EMPTY, 0 IS SET TO INITIAL VALUE OF $\theta$) — S203

VQE

INSTRUCT QUANTUM COMPUTER TO COMPUTE EXPECTED VALUE $\langle \Psi(\theta)|\hat{H}(\mu)|\Psi(\theta)\rangle$ — S204

ACQUIRE COMPUTATION RESULT — S205

UPDATE PARAMETER $\theta$ — S206

UPDATE WIDTH ≤ THRESHOLD? — S207
NO
YES

ACQUIRE ERROR IN THE NUMBER OF ELECTRONS IN OBTAINED QUANTUM STATE $|\Psi(\theta)\rangle$ — S208

ERROR IN THE NUMBER OF ELECTRONS ≤ THRESHOLD? — S209
NO
YES

ADD VALUES OF $(\mu, \theta)$ OBTAINED IN IMMEDIATELY PRECEDING VQE COMPUTATION IN HISTORY LIST — S210

UPDATE VALUE OF $\mu$ — S211

END

European Patent Office
Europäisches Patentamt
European Patent Office
Office européen des brevets

# EUROPEAN SEARCH REPORT

**Application Number**

EP 25 17 5180

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RODOLFO CAROBENE ET AL: "Sequence of penalties method to study excited states using VQE", ARXIV.ORG, CORNELL UNIVERSITY LIBRARY, 201 OLIN LIBRARY CORNELL UNIVERSITY ITHACA, NY 14853, 16 May 2023 (2023-05-16), XP091508738, DOI: 10.1088/2058-9565/ACD1A9 * abstract * * page 1, left-hand column, line 1 - page 7, right-hand column, last line * * page 9, left-hand column, line 1 - page 11, right-hand column, last line * ----- | 1-6 | INV. G06N10/20 G06N10/60 ADD. G06N5/01 G06N20/00 |
| A | HUGH G A BURTON: "Symmetry-preserving and gate-efficient quantum circuits for quantum chemistry", ARXIV.ORG, CORNELL UNIVERSITY LIBRARY, 201 OLIN LIBRARY CORNELL UNIVERSITY ITHACA, NY 14853, 15 December 2023 (2023-12-15), XP091668063, * abstract * * page 2, line 1 - page 15, line 5 * ----- -/-- | 1-6 | |

| | | | TECHNICAL FIELDS SEARCHED (IPC) |
|---|---|---|---|
| | | | G06N |

The present search report has been drawn up for all claims

| Place of search | Date of completion of the search | Examiner |
|---|---|---|
| The Hague | 3 October 2025 | Totir, Felix |

EPO FORM 1503 03.82 (P04C01)

**page 1 of 2**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Le Thinh Viet ET AL: "Variational Quantum Eigensolver with Constraints (VQEC): Solving Constrained Optimization Problems via VQE", arXiv.org, 26 April 2024 (2024-04-26), pages 1-20, XP093312747, Retrieved from the Internet: URL:https://arxiv.org/pdf/2311.08502 [retrieved on 2025-09-10] * abstract * * page 1, left-hand column, line 1 - page 16, right-hand column, last line * * page 18, right-hand column, line 5 - page 20, right-hand column, last line * ----- | 1-6 | |

TECHNICAL FIELDS
SEARCHED (IPC)

The present search report has been drawn up for all claims

| Place of search | Date of completion of the search | Examiner |
|---|---|---|
| The Hague | 3 October 2025 | Totir, Felix |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

**Patent documents cited in the description**

- JP 2023507139 A
- JP 2021504805 A
- US 20210216897
- US 20230143072